# EUROPÄISCHE PATENTSCHRIFT

(11) **EP 0 963 212 B1**
(45) Veröffentlichungstag und Bekanntmachung des Hinweises auf die Patenterteilung: **16.10.2002**
(21) Anmeldenummer: 97918876.0
(22) Anmeldetag: 01.10.1997
(51) Int. Cl.: A61M 5/32

(54) **KANÜLE FÜR MEDIZINISCHE SPRITZEN**
CANNULA FOR MEDICAL SYRINGES
CANULE POUR SERINGUES MEDICALES

(30) Priorität: 23.12.1996 CH 317596
(43) Veröffentlichungstag der Anmeldung: 15.12.1999
(73) Patentinhaber: H. Weidmann AG, 8640 Rapperswil (CH)
(72) Erfinder: BÜTTGEN, Heinz, CH-8645 Jona (CH); CALLENBACH, Tilo, CH-8645 Jona (CH)
(74) Vertreter: Groner, Manfred
(86) Internationale Anmeldenummer: CH9700367
(87) Internationale Veröffentlichungsnummer: WO98028031

(56) Entgegenhaltungen:
- EP-A- 0 287 950
- EP-A- 0 290 176
- US-A- 3 662 457
- US-A- 5 480 385

## Beschreibung

Die Erfindung betrifft eine Kanüle nach dem Oberbegriff des Anspruchs 1.

Eine Kanüle dieser Art ist im Stand der Technik durch die US-5,480,385 bekannt geworden. Bei dieser besteht die Schwierigkeit, dass beim Rückzug der Hohlnadel Injektionslösung, die mit Blut des Patienten vermischt sein kann, durch die Hohlnadel ausgespritzt wird.

Eine ähnliche Kanüle ist im Stand der Technik durch die US-A-5,114,404 bekannt geworden. Bei dieser besteht insbesondere der Nachteil, dass der Nadelhalter und damit die Hohlnadel zur Auslösung gedreht wird. Durch die Drehung der Hohlnadel können im Körper des Patienten Gewebeteile freigesetzt werden, was eine erhebliche Verletzungsgefahr bedeutet. Weiter ist durch die EP-A-0 290 176 eine Kanüle bekannt geworden, die ebenfalls einen Rückzug der Hohlnadel nach ihrer Benutzung ermöglicht. Bei dieser Kanüle besteht jedoch die Schwierigkeit, dass die Hohlnadel um ihre Längsrichtung beliebig orientiert sein kann. Die Orientierung der Nadelspitze zum Kanülengehäuse ist hier somit beliebig und muss vor jeder Injektion zuerst ermittelt werden. Der Rückzug der Hohlnadel erfolgt hier mit einer Druckfeder und es besteht auch hier die Gefahr, dass sich die Hohlnadel bei ihrem Rückzug dreht. Weitere ähnliche Kanülen sind aus der WO 92/16 248 und der WO 92/058 18 bekannt geworden.

Nach der Erfindung sind bei einer gattungsgemässen Kanüle im Kanülengehäuse Mittel für einen Volumenausgleich beim Rückzug der Hohlnadel angeordnet. Diese Mittel sind beispielsweise so ausgebildet, dass vor dem Rückzug ein Unterdruck erzeugt wird. Die beim Rückzug der Hohlnadel verdrängte Flüssigkeit gleicht dann diesen Unterdruck aus. Damit kann vermieden werden, dass beim Rückzug der Hohlnadel Injektionslösung, die mit Blut des Patienten vermischt sein kann, durch die Hohlnadel ausgespritzt wird.

Nach einer bevorzugten Weiterbildung der Erfindung weisen die Mittel für den Volumenausgleich ein im Kanülengehäuse verschiebbar gelagertes Element auf, das zur Erzeugung eines partiellen Unterdrucks vor dem Rückzug der Hohlnadel zu verschieben ist. Dieses verschiebbare Element ist gemäss einer Weiterbildung der Erfindung gleichezeitig ein Auslöselement, mit dem der Rückzug ausgelöst wird. Dies ermöglicht eine Ausführung mit besonders wenigen Einzelteilen.

Nach einer Weiterbildung der Erfindung ist die Hohlnadel an ihrem hinteren Ende am Umfang längsverschieblich geführt und abgedichtet. Damit lässt sich sehr sicher die Gefahr einer Luftinjektion vermeiden. Ebenfalls können Probleme beim Aufzug von Injektionslösung in die Spritze und das Festsetzen von Luftblasen vermieden werden. Da lediglich über den Umfang der Hohlnadel abgedichtet werden muss, ist der Dichtungsbereich sehr klein und ergibt vergleichsweise wenig Reibung beim Rückzug der Nadel. Durch die Führung wird zudem die Hohlnadel stabilisiert. Die Kanüle ist gemäss einer Ausführungsvariante mit einem Luer-Ansatz versehen, was die Verwendung der Kanüle bei einer Standardspritze ermöglicht. Die Kanüle kann aber auch fest an einem Spritzenzylinder angebracht sein.

Eine jederzeitige Auslösung des Rückzuges ist dann möglich, wenn gemäss einer Weiterbildung der Rückzug der Hohlnadel durch radial nach innen und gegeneinander zu bewegende Betätigungselemente auszulösen ist. Diese Betätigungselemente sind vorzugsweise am Kanülengehäuse angeordnet. Durch die gegeneinander gerichteten Bewegungen wird eine ruhige Handhabung und damit eine unnötige Bewegung der Kanüle vermieden.

Weitere vorteilhafte Merkmale ergeben sich aus den abhängigen Patentansprüchen, der nachfolgenden Beschreibung sowie der Zeichnungen.

Ein Ausführungsbeispiel der Erfindung wird nachfolgend anhand der Zeichnungen näher erläutert. Es zeigen:
- Figur 1: Ein Längsschnitt durch eine erfindungsgemässe Kanüle im Grundzustand sowie ebenfalls im Längsschnitt eine Teilansicht eines Spritzenzylinders,
- Figur 2: ein Längsschnitt gemäss Figur 1, jedoch ohne Schutzkappe und nach einem Rückzug der Hohlnadel,
- Figur 3: ein Querschnitt entlang der Linie III-III der Figur 1,
- Figur 4: ein Querschnitt entlang der Linie IV-IV der Figur 1,
- Figur 5: ein Querschnitt entlang der Linie V-V der Figur 1,
- Figur 6: ein Querschnitt entlang der Linie VI-VI der Figur 2, und
- Figur 7: eine Ansicht der erfindungsgemässen Kanüle, jedoch ohne Schutzkappe.

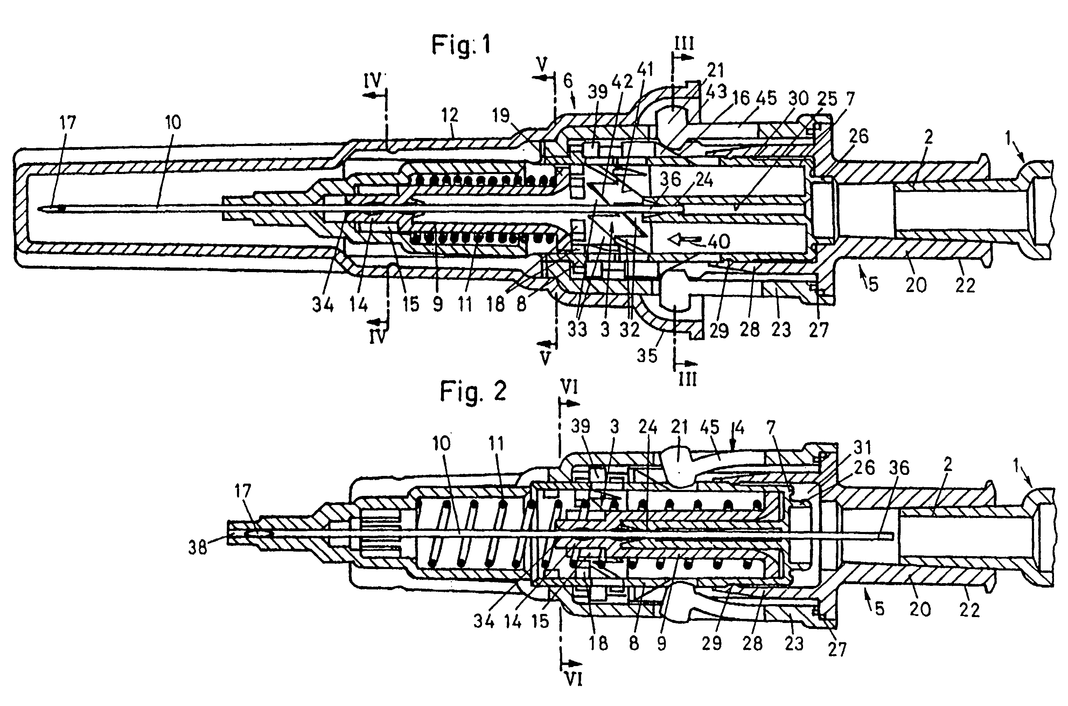

Die in Figur 1 im Grundzustand gezeigte Kanüle 6 ist lösbar auf einen hier nur teilweise gezeigten Spritzenzylinder 1 aufgesetzt. Die Verbindung zwischen der Kanüle 6 und dem Spritzenzylinder 1 besteht aus einem Ansatz 20 am hinteren Ende eines Kanülengehäuses 5 und einem konischen Ansatz 2 am vorderen Ende des Spritzenzylinders 1. Die Verbindungsfläche zwischen den Ansätzen 2 und 20 ist konisch entsprechend den an sich bekannten Luer-Verbindungen. Solche Verbindungen sind bei medizinischen Spritzen gut bekannt. Das Gehäuse 5 kann jedoch gemäss einer hier nicht gezeigten Ausführung auch fest mit dem Spritzenzylinder 1 verbunden sein.

Das Kanülengehäuse 5 besteht aus einem hinteren Gehäuseteil 22 und einem vorderen Gehäuseteil 23, die an einer Verbindungsstelle 27 beispielsweise durch Ultraschall-Schweissung dicht miteinander verbunden sind. Auf das vordere Ende des Gehäuses 5 ist eine Schutzkappe 12 lösbar aufgesetzt, die ein Berühren der Hohlnadel 10 verhindert und die gleichzeitig mit dem hinteren glockenförmigen Rand 35 zwei Betätigungselemente 4 für den Rückzug der Hohlnadel 10 abdeckt.

Die Hohlnadel 10 weist eine übliche scharfe Spitze 17 auf und ist in einem mittleren Bereich mittels einer Klebstelle 34 fest mit einem Nadelhalter 9 verbunden, der an einem hinteren Ende mehrere nach aussen ragende Nocken 19 besitzt, die. an entsprechenden nach innen ragenden Nocken 18 einer Schalthülse 8 abgestützt sind. Eine Druckfeder 11 bildet einen Energiespeicher und spannt den Nadelhalter 9 gegen die genannten Nocken 18. Die Hohlnadel 10 ragt mit ihrem hinteren Ende 36 in einen Längskanal 25 eines Elements 7 und ist an seiner Aussenseite verschieblich mit einer umlaufenden Dichtungslippe 24 abgedichtet. Das Element 7 ist wie nachfolgend näher erläutert sowohl ein Auslöselement als auch ein Volumenausgleichsteil. Das Element 7 ist mit einem hülsenförmigen Ansatz 28 des hinteren Gehäuseteils 22 geführt und mit einer umlaufenden Dichtungslippe 29 abgedichtet sowie mit einem hintergreifenden Wulst 26 in der gezeigten Position fixiert.

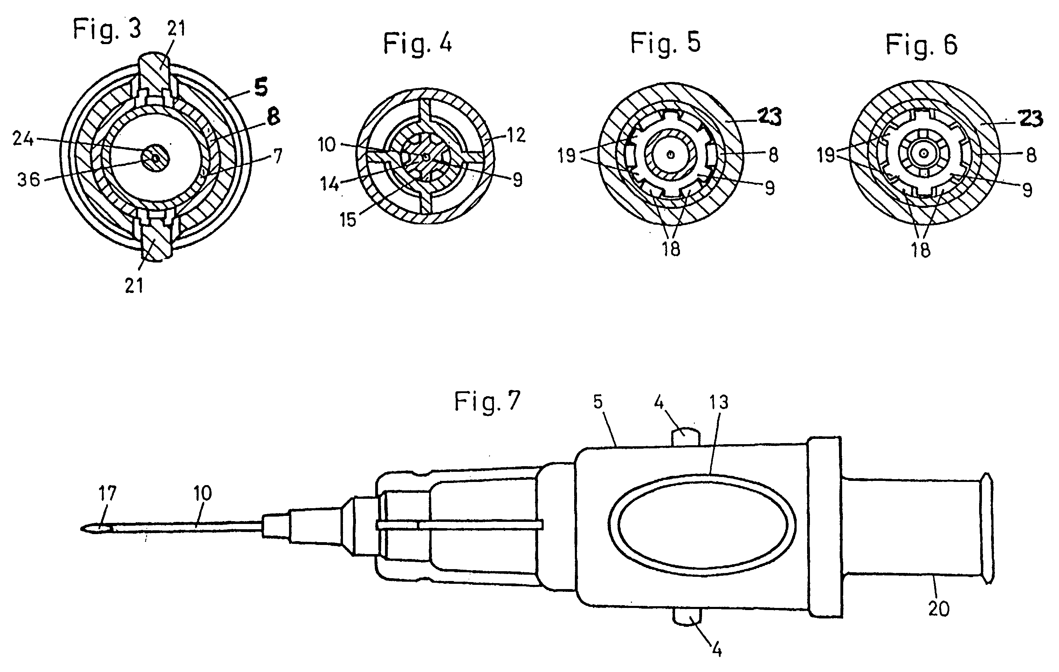

Der Nadelhalter 9 ist in einem vorderen Bereich 14 gemäss Figur 4 mit einem zahnradähnlichen Querschnitt versehen und in einem korrespondierenden Führungskanal 15 des Kanülengehäuses 5 verdrehsicher geführt. Der Nadelhalter 9 ist so in den Führungskanal 15 eingesetzt, dass die schräg angeschliffene Spitze 17 bezüglich der in Figur 7 angedeuteten Markierung 13 immer gleich ausgerichtet ist. Die Orientierung der Spitze 17 ist somit an der Markierung 13 des Kanülengehäuses erkennbar.

Die Hohlnadel 10 ist mit dem Nadelhalter 9 nach einer Benutzung der Kanüle 6 vollständig in das Kanülengehäuse 5 rückziehbar. Der Rückzug erfolgt durch Entspannen der Druckfeder 11. Um den Rückzug auszulösen, wird die Schalthülse 8 gedreht, bis die Nocken 18 gemäss Figur 6 sich jeweils in einer Lücke zwischen den Nocken 19 befinden. Der Nadelhalter 9 ist damit an der Schalthülse 8 nicht mehr abgestützt und wird durch die Druckfeder 11 nach hinten in die in Figur 2 gezeigte Position bewegt. Die Spitze 17 ist hierbei sicher in einem Kanal 38 des Gehäuses 5 untergebracht. Wesentlich ist, dass der Rückzug der Hohlnadel 10 geführt ist und ein Verdrehen zumindest während der ersten Phase des Rückzuges verhindert ist. Beim Auslösen des Rückzugs wird die Schalthülse 8, jedoch nicht der Nadelhalter 9 gedreht. Auch beim Rückzug ist der Nadelhalter 9 während des Eingriffs im Führungskanal 15 gegen ein Verdrehen gesichert. Zudem ist die Hohlnadel 10 während des Rückzugs im Kanal 38 sowie durch die Dichtlippe 24 gestützt.

Die Schalthülse 8 ist ein Teil einer Umschaltvorrichtung 3 und ist an Nocken 39 des Kanülengehäuses 5 drehbar gelagert. Zum Drehen der Schalthülse 8 weist diese rückseitig Schaltzähne 33 auf, die mit korrespondierenden Schaltzähnen 32 des Elementes 7 zusammenarbeiten. Um die Schalthülse 8 zu drehen, wird das Element 7 in Richtung des Pfeils 40 verschoben, wobei schräge Schaltflächen 41 der Zähne 32 mit geneigten Schaltflächen 42 der Zähne 33 in Eingriff gebracht werden. Hierbei wird ein Drehen des Elementes 7 durch radial nach aussen ragende keilförmige Ansätze 43 verhindert. Verschoben wird das Element 7 mit einer Betätigungsvorrichtung, die zwei gegenüberliegende Betätigungselemente 4 aufweist. Diese Betätigungselemente 4 besitzen jeweils einen Betätigungsnokken 21, der mit einem Arm 45 elastisch am vorderen Gehäuseteil 23 angeformt ist. Die Betätigungsnocken 21 werden zum Auslösen des Rückzuges der Hohlnadel 10 von Hand radial nach innen bewegt, und dadurch auf schräge Schaltflächen 16 eine Kraft ausgeübt, welche eine Verschiebung des Elementes 7 in Richtung des Pfeils 40 bewirkt. Bei der Betätigung der Betätigungselemente 4 ist die Schutzhülse 12 selbstverständlich entfernt. Bei aufgesetzter Schutzhülse 12 ist jedoch ein Auslösen des Rückzuges nicht möglich, da die Nocken 21 nicht zugänglich sind.

Beim Verschieben des Elementes 7 in Richtung des Pfeils 40 wird im Kanülengehäuse 5 rückseitig des Elementes 7 ein Volumenausgleichsraum 31 geschaffen, der einen Unterdruck im Kanülengehäuse 5 bewirkt. Damit ist vermieden, dass beim Rückzug der Hohlnadel 10 sich die in dieser befindende Flüssigkeit durch die Spitze 17 ausgestossen wird. Das Element 7 wirkt somit gleichzeitig als Auslöselement als auch als Volumenausgleichsteil. Gleichzeitig dient das Element 7 dazu, die Hohlnadel 10 an ihrem hinteren Ende 36 abzudichten und zu führen.

Nachfolgend wird die Funktionsweise der Kanüle 6 näher erläutert.

Zum Füllen des Spritzenzylinders 1 mit einer Flüssigkeit, insbesondere mit einem Medikament, wird die Schutzkappe 12 vom Kanülengehäuse 5 abgenommen. Die Spitze 17 der Hohlnadel 10 wird in die aufzunehmende Flüssigkeit eingetaucht und der hier nicht gezeigte übliche Kolben des Spritzenzylinders wird entsprechend betätigt, wodurch Flüssigkeit durch die Hohlnadel 10 und durch den abgedichteten Kanal 25 in den Spritzenzylinder 1 strömt. Wie bei Spritzen üblich, kann vor einer Injektion durch einen kurzen Hub des Kolbens Luft ausgestossen werden. Hierbei wird verhindert, dass sich Luftblasen festsetzen und eventuell bei einer Lageänderung lösen können und injiziert werden. Ist der Spritzenzylinder 1 mit der Flüssigkeit gefüllt, so wird die Hohlnadel 10 in üblicher Weise eingeführt, wobei die Markierung 13 die Ausrichtung der Spitze 17 anzeigt. Die Markierung 13 zeigt stilisiert den Nadelanschliff. Die Flüssigkeit wird durch einen Hub des Kolbenzylinders wie üblich injiziert. Nach der Injektion wird von Hand durch radiales Eindrücken der Betätigungselemente 4 der oben erläuterte Rückzug der Hohlnadel 10 ausgelöst. Die Hohlnadel 10 wird hierbei geführt in die in Figur 2 gezeigte Position bewegt, in der eine unbeaufsichtigte Berührung der Hohlnadel 10 nicht mehr möglich ist.

Die Kanüle 6 ist hier im Zusammenhang mit einem Spritzenzylinder 1 gezeigt. Denkbar ist jedoch auch eine Verwendung der Kanüle 6 beispielsweise für die Blutentnahme oder für die In-Vitro-Diagnostik. Bei einer solchen Anwendung kann sich die Entfernung der Kanüle 6 erübrigen. Ausser der Hohlnadel 10 und der Feder 11 können sämtliche übrigen Teile als Spritzgussteile in Kunststoff hergestellt werden. Die Anzahl dieser Teile ist vergleichsweise klein und die Montage eignet sich auch für eine Serienproduktion. Wesentlich ist auch der vergleichsweise kleine Volumenbedarf, so dass sich die erfindungsgemässe Kanüle 6 von einer üblichen Kanüle ohne Nadelrückzug nicht wesentlich unterscheidet.

## Patentansprüche

1. Kanüle mit einer Hohlnadel (10), mit einem an der Hohlnadel (10) angebrachten Nadelhalter (9), der in einem Kanülengehäuse (5) rückziehbar gelagert ist und mit einer eine Umschaltvorrichtung (7, 8) aufweisenden Einrichtung (3), mit der die Hohlnadel (10) nach ihrer Benutzung in das Kanülengehäuse (5) rückziehbar ist, wobei die Umschaltvorrichtung (7, 8) von Hand an der Aussenseite des Kanülengehäuses (5) auslösbar ist und der Nadelhalter (9) die Hohlnadel (10) im Kanülengehäuse (5) in einer vorbestimmten Orientierung gegen ein Verdrehen sichert, **dadurch gekennzeichnet, dass** im Kanülengehäuse (5) Mittel (7) für einen Volumenausgleich beim Rückzug der Hohlnadel (10) angeordnet sind.

2. Kanüle nach Anspruch 1, **dadurch gekennzeichnet, dass** das Kanülengehäuse (5) aussenseitig eine Markierung (13) aufweist, welche auf die Orientierung der Hohlnadel (10) hinweist.

3. Kanüle nach Anspruch 1, **dadurch gekennzeichnet, dass** der Nadelhalter (9) zu seiner Verdrehsicherung einen Bereich (14) mit einem unrunden Querschnitt aufweist, der in einem ebenfalls unrunden Führungskanal (15) des Kanülengehäuses (5) angeordnet ist.

4. Kanüle nach einem der Ansprüche 1 bis 3, **dadurch gekennzeichnet, dass** die Mittel (7) für den Volumenausgleich ein im Kanülengehäuse (5) verschiebbar gelagertes Element (7) aufweisen, das zur Erzeugung eines partiellen Unterdrucks vor dem Rückzug der Hohlnadel (10) zu verschieben ist.

5. Kanüle nach Anspruch 4, **dadurch gekennzeichnet, dass** das Element (7) eine Hülse ist, die beim Auslösen des Rückzugs mit Betätigungsmitteln (15) im Kanülengehäuse (5) verschoben wird.

6. Kanüle nach einem der Ansprüche 1 bis 5, **dadurch gekennzeichnet, dass** die Hohlnadel (10) an ihrem hinteren Ende am Umfang längsverschieblich abgedichtet ist.

7. Kanüle nach Anspruch 6, **dadurch gekennzeichnet, dass** die Hohlnadel (10) gegenüber den im Kanülengehäuse (5) verschieblich gelagerten Volumenausgleichsmitteln (7) abgedichtet ist.

8. Kanüle nach einem der Ansprüche 1 bis 7, **dadurch gekennzeichnet, dass** der Rückzug der Hohlnadel (10) durch radial nach innen und gegeneinander zu bewegende Betätigungselemente (4) auszulösen ist.

9. Kanüle nach Anspruch 8, **dadurch gekennzeichnet, dass** die Betätigungselemente (4) am Kanülengehäuse (5) angeformt sind.

10. Kanüle nach Anspruch 8 oder 9, **dadurch gekennzeichnet, dass** die Betätigungselemente (4) bei ihrer Betätigung ein im Kanülengehäuse (5) gelagertes Auslöselement (7) bewegen.

11. Kanüle nach Anspruch 10, **dadurch gekennzeichnet, dass** das Auslöselement (7) in Längsrichtung der Hohlnadel (10) und nach vorne bewegt wird.

12. Kanüle nach Anspruch 10 oder 11, **dadurch gekennzeichnet, dass** das Auslöselement (7) mit einer Schalthülse (8) zusammenarbeitet, die im Kanülengehäuse (5) gelagert ist und an welcher der Nadelhalter (9) abgestützt ist.

13. Kanüle nach Anspruch 12, **dadurch gekennzeichnet, dass** die Schalthülse (8) drehbar gelagert ist.

14. Kanüle nach Anspruch 12 oder 13, **dadurch gekennzeichnet, dass** die Schalthülse (8) nach innen gerichtete Nokken (18) aufweist, die auf nach aussen gerichteten Nokken (15) des Nadelhalters (19) abgestützt sind.

15. Kanüle nach einem der Ansprüche 1 bis 14, **dadurch gekennzeichnet, dass** auf das Kanülengehäuse (5) eine abnehmbare Schutzkappe (12) aufgesetzt ist, die Betätigungsmittel (4) zum Auslösen des Rückzugs der Hohlnadel (10) wenigstens bereichsweise überdeckt.

16. Kanüle nach einem der Ansprüche 1 bis 15, **dadurch gekennzeichnet, dass** das Kanülengehäuse (5) einen Luer-Ansatz (20) aufweist.

17. Kanüle nach einem der Ansprüche 1 bis 15, **dadurch gekennzeichnet, dass** die Kanüle fest an einem Spritzenzylinder (1) angebracht ist.

## Claims

1. A syringe comprising a hollow needle (10), needle holder (9) attached to the needle (10) whereby the needle holder (9) is located in the syringe housing (5) where it may be retracted, a device (3) that has a switching mechanism (7, 8) by which the hollow needle (10) may be retracted into the syringe housing (5), wherein the switching mechanism (7, 8) may be triggered by hand at the exterior side of the syringe housing (5) and that the needle holder (9) keeps the needle (10) from turning in the syringe housing (5) and maintains the needle (10) in a predetermined position, **characterized in that** there are means inside the syringe housing (5) for volume compensation during retraction of the hollow needle (10).

2. A syringe according to claim 1, **characterized in that** the syringe housing (5) has on its exterior side a marking (13), which shows the position of the needle (10).

3. A syringe according to claim 1 and 2, **characterized in that** the needle holder (9) has a section (14) that has a non-circular cross section, which is also located in a non-circular guiding channel (15) within the syringe housing (5).

4. A syringe according to one of the claims 1 through 3, **characterized in that** the means (7) for volume compensation have a movable element (7) located within the syringe housing (5), which may be moved to create a partial vacuum before retraction of the needle (10).

5. A syringe according to claim 4, **characterized in that** the element (7) is a shell-shaped device that is moved within the syringe housing (5) during triggering of retraction by an actuating mechanism (15).

6. A syringe according to one of the claims 1 through 5, **characterized in that** the needle (10) has a seal around its circumference and maintains said seal when moved longitudinally.

7. A syringe according to claim 6, **characterized in that** the needle (10) is sealed off from a movable volume-compensation mechanism (7) located within the syringe housing (5).

8. A syringe according to claims 1 through 7, **characterized in that** retraction of the needle (10) is triggered by actuating elements (4), which move in a circle toward the inside and against one another.

9. A syringe according to claim 8, **characterized in that** the actuating element (4) are molded to the syringe housing (5).

10. A syringe according to claim 8 or 9, **characterized in that** the actuating elements (4) move a trigger element (7) during operation, which is located in the syringe housing (5).

11. A syringe according to claim 10, **characterized in that** the trigger element (7) is moved forward in the direction of the needle (10).

12. A syringe according to claim 10 or 11, **characterized in that** the trigger element (7) operates in conjunction with a switching box (8) that is located inside the syringe housing (5) and that the needle holder (9) is supported by said switching box (8).

13. A syringe according to claim 12, **characterized in that** the switching box (8) is mounted in a manner where it may be rotated.

14. A syringe according to claim 12 or 13, **characterized in that** the switching box (8) has projection (18) facing inwardly and whereby said projection (18) rest against the projections (15) of the needle holder (19), which are facing the outside.

15. A syringe according to one of the claims 1 through 14, **characterized in that** a detachable protective cap (12) is placed on the syringe housing (5) and whereby said protective cap (12) covers for the most part the actuating means (4) that trigger retraction of the needle (10).

16. A syringe according to one of the claims 1 through 15, **characterized in that in that** the syringe housing has a Luer nozzle (20).

17. A syringe according to one of the claims 1 through 15, **characterized in that** the syringe is rigidly attached to a barrel (1).

## Revendications

1. Canule comprenant une aiguille d'injection (10), un porte-aiguille (9), qui est monté contre l'aiguille d'injection (10) et est logé de manière rétractable dans un corps de canule (5), et une unité (3) munie d'un système inverseur (7, 8), par lequel l'aiguille d'injection (10) se rétracte après usage dans le corps de canule (5), sachant que le système inverseur (7, 8) est déclenché à la main sur le côté extérieur du corps de canule (5) et le porte-aiguilles (9) fait en sorte que l'aiguille d'injection (10) reste dans une orientation prédéfinie évitant une torsion à l'intérieur du corps de canule (5), **caractérisée en ce que** des moyens (7) destinés à équilibrer le volume au moment du mouvement de rétraction de l'aiguille d'injection (10) sont disposés dans le corps de canule (5).

2. Canule selon la revendication 1, **caractérisée en ce que** la face extérieure du corps de canule (5) est munie d'un repère (13), qui indique l'orientation de l'aiguille d'injection (10).

3. Canule selon la revendication 1, **caractérisée en ce que** le porte-aiguille (9) pour empêcher un mouvement de torsion est muni d'une zone (14) avec une section ovalisée, qui est disposée dans un canal de guidage (15), également ovalisé, dans le corps de canule (5).

4. Canule selon une des revendications 1 à 3, **caractérisée en ce que** les moyens (7) destinés à équilibrer le volume comportent un élément (7), qui est logé de manière à pouvoir coulisser dans le corps de canule (5) et qu'il faut faire coulisser pour générer une dépression partielle avant le mouvement de rétraction de l'aiguille d'injection (10).

5. Canule selon la revendication 4, **caractérisée en ce que** l'élément (7) est une gaine, que des moyens de commande (15) font coulisser dans le corps de canule (5) au moment du déclenchement du mouvement de rétraction.

6. Canule selon une des revendications 1 à 5, **caractérisée en ce que** le pourtour de l'aiguille d'injection (10) est rendu étanche au niveau de son extrémité arrière pour coulisser dans le sens longitudinal.

7. Canule selon la revendication 6, **caractérisée en ce que** l'aiguille d'injection (10) est rendue étanche par rapport aux moyens d'équilibrage du volume (7), logés de manière à pouvoir coulisser dans le corps de canule (5).

8. Canule selon une des revendications 1 à 7, **caractérisée en ce que** le mouvement de rétraction de l'aiguille d'injection (10) est déclenché par des éléments de commande (4) à déplacer dans le sens radial vers l'intérieur et l'un par rapport à l'autre.

9. Canule selon la revendication 8, **caractérisée en ce que** les éléments de commande (4) sont formés contre le corps de canule (5).

10. Canule selon la revendication 8 ou 9, **caractérisée en ce que** les éléments de commande (4), au moment de leur actionnement, déplacent un élément de déclenchement (7) logé à l'intérieur du corps de canule (5).

11. Canule selon la revendication 10, **caractérisée en ce que** l'élément de déclenchement (7) est déplacé dans le sens longitudinal de l'aiguille d'injection (10) et vers l'avant.

12. Canule selon la revendication 10 ou 11, **caractérisée en ce que** l'élément de déclenchement (7) coopère avec une gaine de couplage (8), qui est logée dans le corps de canule (5) et sur laquelle le porte-aiguille (9) est en appui.

13. Canule selon la revendication 12, **caractérisée en ce que** la gaine de couplage (8) est logée de manière rotative.

14. Canule selon la revendication 12 ou 13, **caractérisée en ce que** la gaine de couplage (8) est munie de tétons (18) orientés vers l'intérieur, qui sont en appui sur des tétons (15) du porte-aiguille (9), orientés vers l'extérieur.

15. Canule selon une des revendications 1 à 14, **caractérisée en ce qu'**un capuchon de protection (12) amovible est posé sur le corps de canule (5) et recouvre au moins en partie les moyens de commande (4) destinés à déclencher le mouvement de rétraction de l'aiguille d'injection (10).

16. Canule selon une des revendications 1 à 15, **caractérisée en ce que** le corps de canule (5) est muni d'un embout Luer (20).

17. Canule selon une des revendications 1 à 15, **caractérisée en ce que** la canule est assemblée fermement avec une seringue cylindrique (1).
